(19)

**Europäisches Patentamt**

**European Patent Office**

**Office européen des brevets**

(11)  **EP 4 550 264 A1**

(12)  # EUROPEAN PATENT APPLICATION

(43) Date of publication:
07.05.2025  Bulletin 2025/19

(21) Application number: 23207342.9

(22) Date of filing: 02.11.2023

(51) International Patent Classification (IPC):
$G06T\ 7/33^{(2017.01)}$       $G06T\ 7/00^{(2017.01)}$
$A61B\ 6/03^{(2006.01)}$       $A61B\ 6/50^{(2024.01)}$

(52) Cooperative Patent Classification (CPC):
A61B 6/035; A61B 6/482; A61B 6/503;
A61B 6/504; A61B 6/5217; G06T 7/0012;
G06T 7/33

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Koninklijke Philips N.V.
5656 AG Eindhoven (NL)

(72) Inventors:
• BONDAR, Maria Luiza
  Eindhoven (NL)
• HEESE, Harald Sepp
  Eindhoven (NL)
• ISOLA, Alfonso Agatino
  5656AG Eindhoven (NL)
• NICKISCH, Hannes
  Eindhoven (NL)

• NEUKIRCHEN, Christoph
  Eindhoven (NL)
• PETERS, Jochen
  Eindhoven (NL)
• WIEMKER, Rafael
  Eindhoven (NL)
• BROSCH, Tom
  Eindhoven (NL)
• BONTUS, Claas
  Eindhoven (NL)
• MATUTE FLORES, Jose Alejandro
  Eindhoven (NL)
• JAYARAJ, Ajayraj
  Eindhoven (NL)

(74) Representative: Philips Intellectual Property &
Standards
High Tech Campus 52
5656 AG Eindhoven (NL)

(54)  **DEVICE AND METHOD FOR IMAGE PROCESSING OF SPECTRAL IMAGES OF A SUBJECT**

(57)  The present invention relates to a device and method for image processing of spectral images of a subject. The device comprises an input unit configured to obtain two spectral image data sets of a region of interest, ROI, of the subject acquired at different points in time, each spectral image data set comprising two or more spectral images of the ROI in two or more different channels, the ROI including coronaries. A processing unit is configured to perform a first alignment of the two spectral image data sets by use of image registration; identify one or more corresponding coronary segments in the aligned spectral image data sets; perform a second alignment of the two spectral image data sets, which is a finer alignment than the first alignment, by use of multichannel image registration of the one or more corresponding coronary segments; and determine, per channel, image differences between the finer aligned spectral image data sets. An output unit is provided to output the finer aligned spectral images of the channel having the largest image differences.

FIG.3

EP 4 550 264 A1

**Description**

FIELD OF THE INVENTION

The present invention relates to a device and method for image processing of spectral images of a subject, such as a patient or other person.

BACKGROUND OF THE INVENTION

Coronary artery disease (CAD) is a progressive disease. To monitor disease progression for a patient, coronary computed tomography angiography (CCTA) scans might be acquired at multiple points in time. In addition, multiple CCTA scans might be acquired for a patient follow-up, e.g. after the implantation of stents. Changes and deformation of coronaries can occur between CCTA acquisition time points, for instance due to arterial wall remodeling, disease progression, differences in patient pose, acquisition time during cardiac cycle, etc. For the user, it is difficult to establish a correspondence between segments (slices) of the coronaries in CCTA images acquired at different time points. In addition, CCTA images acquired in dual energy mode bring additional complexities due to the large number of available spectral result images, for instance, several virtual monoenergetic images, iodine map, effective atomic number map (Z_effective), conventional, virtual non-contrast, etc.

Generally, the problem of spatial correspondence can be resolved using image registration techniques. Nevertheless, applying image registration directly on the 3D CCTA image volumes is likely to focus on aligning large structures (heart and lungs) and have less focus on the smaller coronary trees. The large amount of spectral CT images increases the complexity of the correspondence problem, for instance, choice of image pairs to register, number of image pairs to register and the complexity of the task of reviewing the image registration results.

SUMMARY OF THE INVENTION

It is an object of the present invention to improve the alignment of spectral image data sets, in particular to provide a correspondence between segments or slices of the coronaries in spectral image data sets, e.g. in CCTA images, acquired at different time points.

In a first aspect of the present invention a device for image processing of spectral images of a subject is presented, the device comprising:

- an input unit configured to obtain two spectral image data sets of a region of interest, ROI, of the subject acquired at different points in time, each spectral image data set comprising two or more spectral images of the ROI in two or more different channels, the ROI including coronaries;

- a processing unit configured to

  - perform a first alignment of the two spectral image data sets by use of image registration;
  - identify one or more corresponding coronary segments in the aligned spectral image data sets;
  - perform a second alignment of the two spectral image data sets, which is a finer alignment than the first alignment, by use of multi-channel image registration of the one or more corresponding coronary segments; and
  - determine, per channel, image differences between the finer aligned spectral image data sets; and

- an output unit configured to output the finer aligned spectral images of the channel having the largest image differences.

In a further aspect of the present invention a corresponding method for image processing of spectral images of a subject is presented.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention provides a tool to establish a spatial correspondence between regions of the coronaries in spectral image data sets acquired at different time points. The spectral image data sets may e.g. be spectral CT image data sets, in particular CCTA image data sets, and may each comprise two or more of a spectral base image, a monoE40KeV image, an iodine map image, and a Z_effective image, which represent examples of the different channels.

The present invention is based on the idea to apply multi-channel image registration to coronary segments identified in initially aligned spectral image data sets, in particular - as provided in an embodiment - in image volumes extracted from a (straightened) curved planar reformation of coronaries in longitudinal spectral CT image series. The user, e.g. a physician or other medical practitioner, is thus able to correlate segments and/or slices and/or regions of coronaries in two or more images (e.g. CT images) and to determine changes in disease status with more confidence.

This approach may be further improved in an embodiment by indicating which of the multiple channels contains the most significant display of anatomical changes by means of analyzing inter-channel variations of the residual image differences after registration.

In an embodiment of the present invention, the alignment of two spectral image data sets is performed by simultaneous alignment of image pairs, i.e., of two images of the same channel but acquired at different time points. Through the alignment a single alignment transformation or vector field is preferably determined. The measure or metric of alignment can be different for the different image pairs, e.g., the image similarity metric for iodine images pair can be different from the image similarity metric for mono-energy images. Different transformations for different image pairs (i.e., image pairs of different channels) are not desired because the transformation finds correspondences between two anatomies imaged at two different points in time (i.e., the same body area such as, e.g., heart or coronaries, image at different times). Each anatomy is described by a series of spectral images. Since there are only two anatomies, a unique global transformation is preferably determined from all image pairs. There may be various implementation methods to determine the unique transformation. An exemplary implementation may follow an iterative approach where the global transformation is initialized with the result of the transformation that aligns two image pairs. The inspection of the alignment may be done by inspecting image pairs.

According to an implementation the processing unit is further configured to generate straightened curved planar reformations for the coronary segments identified in the aligned spectral image data sets; and perform the second alignment of the two spectral image data sets by use of multi-channel image registration of the straightened curved planar reformations of the one or more corresponding coronary segments.

The processing unit may further be configured to perform the multi-channel image registration by minimizing an energy function defined by a weighted sum of similarity measures of each channel. This represents a simple but efficient way of performing the multi-channel image registration.

Hereby, in an embodiment an individual weight may be assigned to each channel for computing the weighted sum. Weights may be used that are predetermined for each channel, that are set or modified by a user, or that are automatically computed, in particular by an automatic iterative weights tweaking method. In such an embodiment several points may be computed on a Pareto front which converge to a solution where the objective functions may have equal contribution (balance solution).

To further improve the accuracy of the result of the proposed method, the processing unit may be further configured to use an individual similarity measure for each channel for computing the weighted sum. Hereby, a similarity measure may be used for each channel selected from a group of similarity measures comprising normalized cross correlation, mutual information metric, information-based similarity measures, geometry-based similarity measures, sum of squared intensity differences, ratio image uniformity, and one or more derivates of a combination of one or more of these metrics.

According to another embodiment, the processing unit is further configured to perform the second alignment of the two spectral image data sets multiple times by performing the multi-channel image registration using different combinations of weights and/or similarity measures; and determine for each of the second alignments, per channel, image differences between the finer aligned spectral image data sets, wherein the output unit is configured to output, for each of the second alignments, the finer aligned spectral images of the channel having the largest image differences. This will further improve the accuracy of the proposed method.

According to another embodiment, the processing unit is further configured to determine, by minimizing the energy function, a deformation model or transformation between spectral images of the same channel from different spectral image data sets, in particular a deformation model or transformation that is inverse consistent or invertible. The deformation model or transformation can be determined such that it has certain desirable properties, e.g., such that the transformation is invertible meaning that the transformation does not fold or self-intersect the space. To make the transformation invertible extra regularization terms may be added in the energy function.

In an implementations, the processing unit is configured to determine, per channel, image differences between the finer aligned spectral image data sets by use of a difference metric qualifying residual image intensity differences. This difference metric may be a sum of residual differences, a maximum, an x-th percentile, number of voxels in one or more regions defined by connected voxels with image difference above a threshold value, and one or more derivates of a combination of one or more of these metrics.

In another implementation, the processing unit is further configured to extract coronary centerlines in the first aligned spectral image data sets; identify the one or more corresponding coronary segments in the extracted coronary centerlines; and generate straightened curved planar reformations for the coronary segments using the extracted coronary centerlines and the original non-reformatted image data sets.

BRIEF DESCRIPTION OF THE DRAWINGS

These and other aspects of the invention will be apparent from and elucidated with reference to the embodiment(s) described hereinafter. In the following drawings:

Fig. 1 shows a schematic diagram of an embodiment of a system according to the present invention.

Fig. 2 shows a schematic diagram of an embodiment of a device according to the present invention.

Fig. 3 shows a flow chart of a first embodiment of a method according to the present invention.

Fig. 4 shows a flow chart of a second embodiment of a method according to the present invention.

Fig. 5 shows a diagram illustrating the first four steps of the method illustrated in Fig. 4.

Fig. 6 shows a diagram illustrating straightened curved planar reformations for two series of spectral images.

Fig. 7 shows a diagram illustrating image differences of straightened curved planar reformations in different channels.

Fig. 8 shows a diagram illustrating an exemplary transformation and the correlation between two straightened coronary cylinders.

DETAILED DESCRIPTION OF EMBODIMENTS

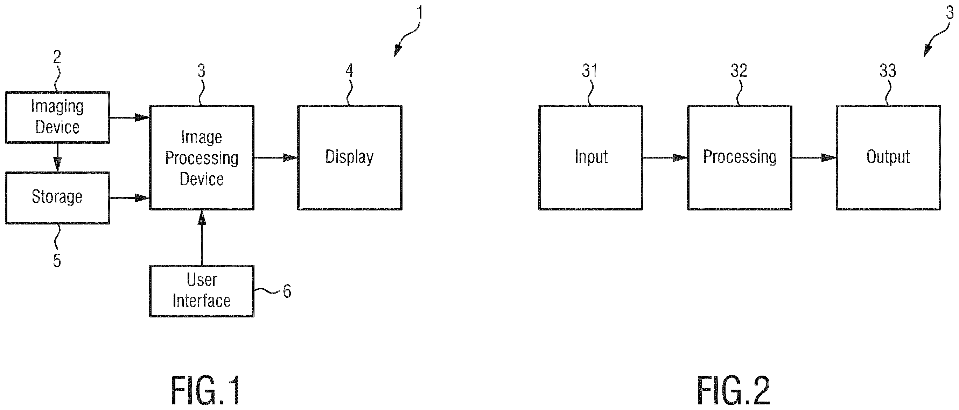

Fig. 1 shows a schematic diagram of an embodiment of an imaging system 1 for generating and processing medical images of a region of interest (ROI) of a subject according to the present invention. The system 1 comprises an imaging device 2 for acquiring spectral image data sets of an ROI of a subject, each spectral image data set comprising two or more spectral images of the ROI in two or more different channels, the ROI including coronaries. The system 1 further comprises a device 3 for image processing of the acquired spectral images of the subject and a display unit 4 configured to display particular images obtained by the image processing of the device 3, in particular finer aligned spectral images of the channel having the largest image differences.

The imaging device 2 is a CT device, in particular a spectral CT or photon-counting CT device, e.g. a conventionally used CT device for acquiring image data of a subject, in particular CCTA images showing coronaries. The imaging device acquires spectral image data sets at different points in time, e.g. before and/or during and/or shortly after an intervention and/or later during one or more checkups. Each spectral image data set comprises two or more spectral images in different channels. For instance, the spectral image data set may comprise one or more base images, a monoE40KeV image (or other monoenergetic image or another energy, e.g. 60KeV, 80KeV, etc.), a full-energy image, an iodine map, a Z_effective (also called Zeff) image, etc., which may be acquired simultaneously or subsequently (almost simultaneously), depending on the implementation of the imaging device, in particular its detector and its radiation source.

The device 3 may e.g. be implemented as or in a processor or computer, in software and/or hardware. For instance, a programmed processor may be included in the device 3, which may execute a computer program that may be stored in a memory that is accessed by the processor.

The display unit 4 may e.g. be a monitor or screen of a PC, workstation, laptop tablet, etc., e.g. of a computer that represents the device 3 and is carrying out the method according to the present invention.

The device 3 may directly obtain (i.e. retrieve or receive) the current spectral image data set directly from the imaging device 2 or from a storage 5, such as a buffer or memory or image repository, e.g. a patient record stored in a hospital's document management system, e.g. via a wired or wireless network. Spectral image data sets acquired earlier are preferably obtained from a storage 5, such as a buffer or memory or image repository A user interface 6, e.g. a touch screen, pointer, computer mouse, etc., may be provided so that a user can provide user input, for instance to interactively mark or correct the ROI or sub-ROI(s) in at least one of two or more spectral images of the spectral image data set or to navigate through spectral images or even modify one or more parameters used during the image processing.

Fig. 2 shows a schematic diagram of an embodiment of a device 3 for image processing of spectral images according to the present invention. The device may comprise circuitry, e.g. a processor, processing circuitry, a computer, dedicated hardware, etc. that carries out the functions of the device. In another embodiment, as shown in Fig. 2, separate units or elements may be used that together represent circuitry of the device 3.

In this embodiment the device 3 comprises an input unit 31 configured to obtain two spectral image data sets of an ROI of the subject acquired at different points in time. he spectral image data set may be obtained, e.g. from the imaging device 2 or the storage 5. For this purpose the input unit 31 may be directly or indirectly (e.g. via a network or bus) coupled or connected to the imaging device 2 or the storage 5. The input unit may thus e.g. be a (wired or wireless) communication interface or data interface, such as a Bluetooth interface, Wi-Fi interface, LAN interface, HDMI interface, direct cable connection, or any other suitable interface allowing data transfer to the device 3.

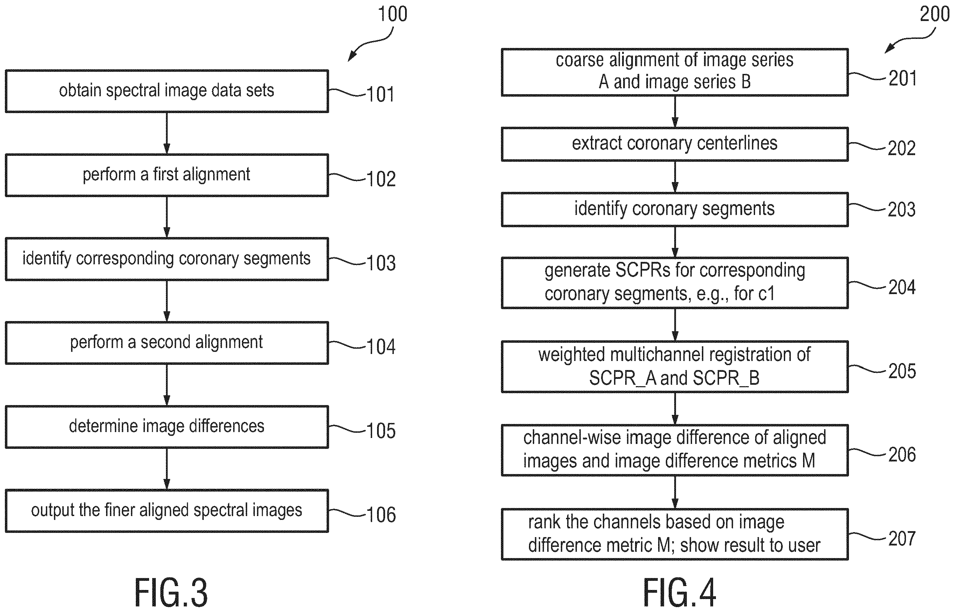

The device 3 further comprises a processing unit 32 configured to carry out the steps of the method according to the present invention as explained below with reference to Fig. 3. The processing unit 32 may be any kind of means configured to process the obtained spectral image data sets and to generate aligned spectral images and/or spatial correspondences between coronaries in spectral images, in particular to generate, per channel, image differences between finer aligned spectral image data sets so that finer aligned spectral images of the channel having the largest image differences can be outputted. The processing unit 32 may be implemented in software and/or hardware, e.g. as a programmed processor, computer, laptop, PC, workstation, etc.

The device 3 further comprises an output unit 33 configured to output finer aligned spectral images of the channel having the largest image differences and, optionally, other information or images obtained by the image processing of the spectral image data sets. The output unit 33 may generally be any interface that provides the generated visualization, e.g. transmits it to another device or provides it for retrieval by another device, e.g. transmits it to another computer or transfers it directly to the display 4 for displaying it. It may thus generally be any (wired or wireless) communication or data interface.

Fig. 3 shows a flow chart of a first embodiment of a method 100 for image processing according to the present invention. The steps of the method may be carried out by the device 3, wherein the main steps of the method are carried out by the processing unit 32. The method may e.g. be implemented as computer program running on a computer or processor.

In a first step 101 two spectral image data sets of an ROI of the subject acquired at different points in time are obtained. In a second step 102 a first alignment of the two spectral image data sets is performed by use of image registration. In a third step 103 one or more corresponding coronary segments are identified in the aligned spectral image data sets. In a fourth step 104 a second alignment of the two spectral image data sets is performed. This second alignment is a finer alignment than the first alignment and is performed by use of multi-channel image registration of the one or more corresponding coronary segments. In a fifth step 105 image differences between the finer aligned spectral image data sets are determined per channel. In a sixth step 106 the finer aligned spectral images of the channel having the largest image differences are outputted.

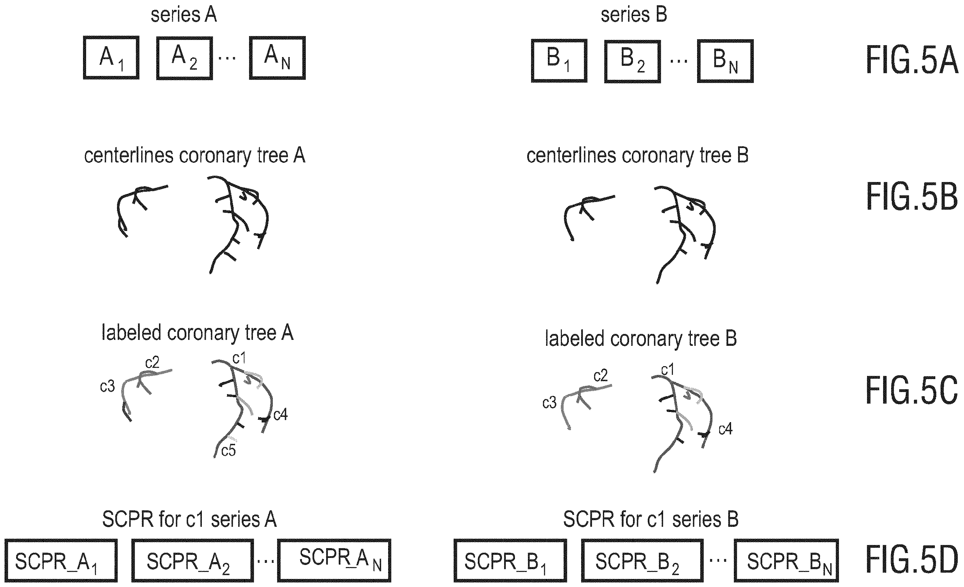

Fig. 4 shows a flow chart of a second embodiment of a method 200 for image processing according to the present invention, which may be carried out by the device 3, in particular the processing unit 32 as well. The method 200 may be regarded as an exemplary implementation of the method 100. Figs. 5 to 7 illustrate some of the steps of this method.

In a first step 201, the spectral image volumes of two spectral image series are coarsely aligned, e.g., using standard 3D image registration methods (e.g., rigid, affine, non-rigid image registration methods). For instance, as shown in Fig. 5A, the two image series A (comprising N spectral images $A_1$ to $A_N$) and series B (comprising N spectral images $B_1$ to $B_N$) are aligned.

In a second step 202, coronary centerlines are extracted from the aligned series A and B. Exemplary centerlines of a coronary tree A and a coronary tree B for the respective series A and B are shown in Fig. 5B.

In a third step 203, corresponding coronary segments are identified in the aligned centerlines for series A and B. Hereby, start and end of a coronary segment may be defined by branching points. The corresponding coronary segments may be labelled by corresponding labels as shown in Fig. 5C.

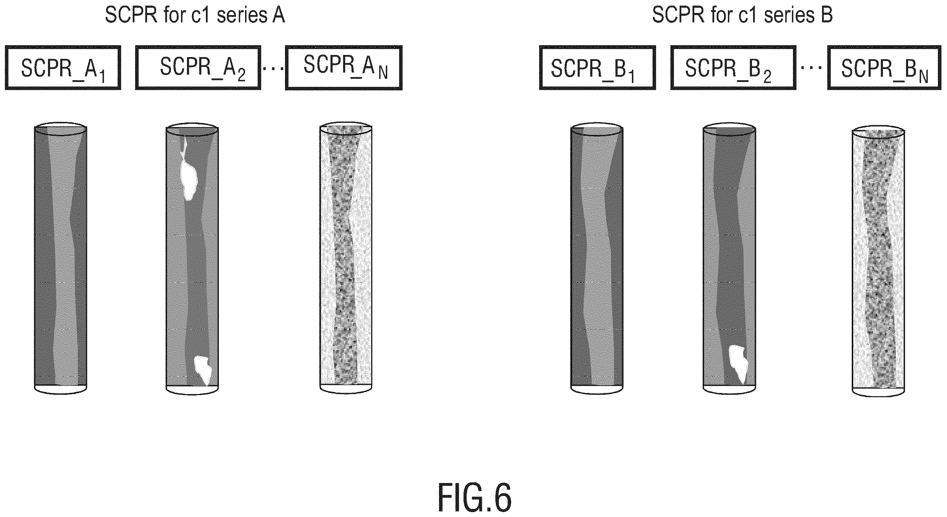

In a fourth step 204, for each coronary segment a straightened curved planar reformation (SCPR), i.e., SCPR_A and SCPR_B, is generated. For instance, as shown in Fig. 5D, for the segment indicated by label c1 in Fig. 5C, $SCPR\_A_1$ to $SCPR\_A_N$ and $SCPR\_B_1$ to $SCPR\_B_N$ are generated. Fig. 6 shows exemplary SCPRs for $SCPR\_A_1$ to $SCPR\_A_N$ and $SCPR\_B_1$ to $SCPR\_B_N$.

In a fifth step 205, a multi-channel image registration is performed to align SCPR_A and SCPR_B. The multi-channel image registration represents a finer alignment of the two spectral image data sets than the initial coarse alignment in step 201. It may optionally be user-guided and/or may optionally use weights in the registration. A transformation or deformation vector field v is thus obtained.

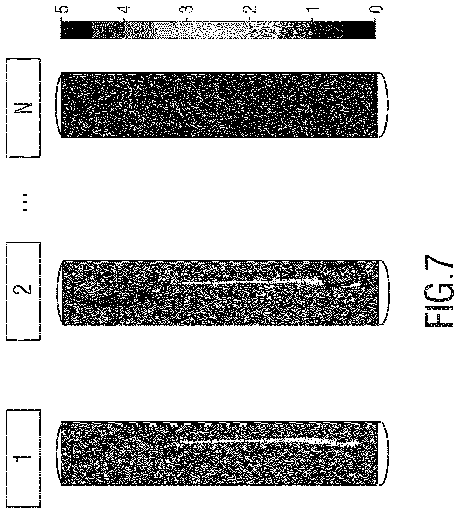

In a sixth step 206, channel-wise image difference of registration results on identified coronary segments are calculated. For instance, a difference metric M indicating the image differences between images of the image series within the same channel are determined. For instance, the difference in channel i may be obtained as $SCPR\_A_i - SCPR\_B_i \circ v$, and the difference metric in channel i may be written as $M(SCPR\_A_i - SCPR\_B_i \circ v)$. Fig. 7 show, per channel of the channels 1 to N, the channel-wise image differences, wherein the different grey values indicate the magnitude of the difference.

In a seventh step 207, the aligned SCPR_A and SCPR_B are presented to the user, preferably in a graphical user interface, selecting the channel with largest channel-wise difference. Hereby, as metric the sum of volumes of the largest three connected components may be used. As an example, channel 2 may thus be selected as the channel showing the larges image differences.

Optionally, in an additional step (not shown) a Pareto front navigator may be used to support the user, such as a clinical expert, to move / navigate over different combinations of weights $w_i$ of multi-channel image registration and to receive the corresponding approximated (expected) image registrations as GUI output. Interpolation of the Pareto front can be performed to allow some fast and real-time navigation. The Pareto front approximation may require running a limited number of registrations with some properly chosen sets of weights. To perform a registration, a deformation model (or a transformation) and an image similarity metric $S_i$ may be used as described below in more detail.

Pareto front navigators are generally known in the art. The weights of the energy function reflect the relative importance of each objective. By varying the weights and minimizing the energy function for the varying weights different points on the Pareto front can be obtained. A point on the Pareto front is defined as a single objective optimization of the energy function for fixed, given value of weights. The weights are changed systematically, and each different single energy function optimization determines
a different optimal solution. The solutions obtained approximate the Pareto front. Pareto front navigation is the process of exploring the Pareto front and finding solutions that match a criterion. A Pareto Front is, for instance, described in Kim, I. Y.; de Week, O. L. (2005). "Adaptive weighted sum method for multiobjective optimization: a new method for Pareto front generation". Structural and Multidisciplinary Optimization. 31 (2): 105-116.

Multi-channel image registration performed in steps 104 and 205, respectively, can be mathematically formulated as, for example, the minimization of an energy function defined as a weighted sum of similarity measures of each channel. For a moving image series with N channels, i.e., moving image series is $\{M_1, M_2, ... , M_N\}$, and a fixed image series with the same number of channels, i.e., $\{F_1, F_2, ... , F_N\}$, the energy function may be defined as

$$E = \sum_{i=1}^{N} w_i\, S_i(M_i \circ \mathbf{v}, F_i),$$

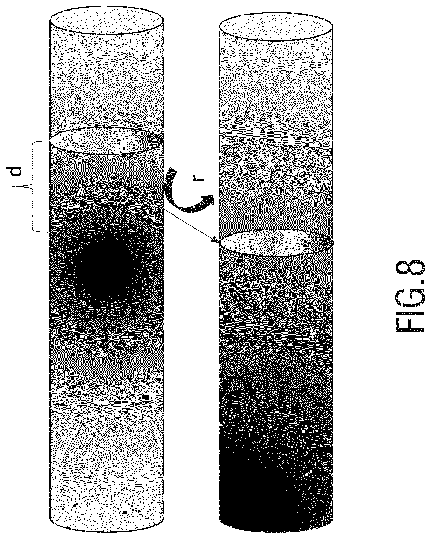

where $w_i > 0$ is a weight that determines the contribution of each channel i to the final energy function, $S_i$ is the similarity measure for the channel, and v is a transformation (or deformation model) such that the transformed moving images are aligned with the target images. Fig. 8 shows an exemplary transformation between two coronary segments schematically shown as cylinders, where the transformation includes a translation $d$ and a rotation $r$.

The multi-channel image registration thus provides that the moving images can be warped into the fixed images. In other words, if $x$ is the physical coordinate of a voxel in a fixed image $F$, $x + v$ gives the physical coordinate of the corresponding voxel in image $M$. The aim of image registration is to find correspondences between voxels in the fixed image and the moving image.

The weights $w_i$ can be determined from registration experiments of previous patients, or user guided (e.g., the user may set weights for a channel to 0, or increase / decrease the weight for a channel). The user may select a list of channels to register. In the GUI, the user may be presented with a prefilled list of channels and/or weights, in which the user may be able to modify the value of the weights and/or the list of channels. Table 1 shows such a prefilled list.

Table 1

| moving | fixed | weight |
| --- | --- | --- |
| MonoE40 | MonoE40 | 10 |
| MonoE120 | MonoE120 | 50 |
| Conventional | Conventional | 20 |

In another embodiment, image similarity measures S, are normalized image similarity measures to the same codomain [0,1] (as e.g. described in Bardera, A.,

Feixas, M., and Boada, I., "Normalized similarity measures for medical image registration", in Medical Imaging 2004: Image Processing, 2004, vol. 5370, pp. 108-118). The codomain of a function is the set of values that a function can take. The weights are automatically calculated by an automatic iterative weights tweaking method toggling between the optimal search of the best registration deformation field v (i.e., minimizing E with fixed $w_i$), and the search of optimal weights $w_i$ (at fixed deformation). For example, the algorithm finds the optimal weights minimizing the ratio between each single metric value and the average metrics mean $S_i$ value.

An iterative weight tweaking method may thus be used as a possible extension or variant of the last step of using a Pareto front navigator to support the user to move / navigate over different combinations of weights. In such a variant several points may be computed on the Pareto front which converge to a solution where the objective functions $S_i$ may have equal contribution (balance solution). Such an iterative weight tweaking algorithm may comprise an initialization to obtain an initial solution with a given set of weights and iterations of steps of scaling the weights using current values of individual functions, e.g., average values, and re-optimization.

Generally, if O is a cost function defined as a weighted sum of m objective functions where $w_i>0$ is a weight and $c_i$ is a cost function, i.e.,

$$O(t) = \sum_{i=1}^{m} w_i c_i(t)$$

let $t^+ = argmin_t\, O(t)$ be the new solution at current iteration $n$-th, then the average constraints value is computed as:

$$\bar{c}^{(n)}(t^+) = \frac{1}{m}\sum_{i=1}^{m} c_i^{(n)}(t^+)$$

The weights are updated using the following formula:

$$w_i^{(n+1)} = \begin{cases} w_i^{(n)}, & if\ \ \bar{c}^{(n)}(t^+) = 0 \\ w_i^{(n)} \cdot \dfrac{c_i^{(n)}(t^+)}{\bar{c}^{(n)}(t^+)}, & otherwise. \end{cases}$$

Examples of a deformation model are described next. A coronary segment centerline is a 3D curve, mathematically represented as C(t) = {X(t), Y(t), Z(t), t∈[0, L]}, where L is the length of the curve and X(t), Y (t), Z(t) are the 3D coordinates of the curve. In addition, for the SCPR representation, an angle θ(t) can be associated for each point on the curve. The angle θ(t) is the sum of the angle of the user-specific direction in the first frame of the coronary image cylinder and the angle determined by rotation-minimizing frames. For a fixed image cylinder volume determined by the centerline $C_f$ segment and a moving image cylinder determined by $C_m$ the deforma-

tion model relating the two image cylinders consists of a shift function d(t) and a rotation r(t) (see Fig. 8) such that $t_m$ corresponds to $t_f = d(t_m)$ and the angle $\theta_m(t_m)$ corresponds to $\theta_f(t_f) + r(t_m)$. For the registration of the two cylinders, regularization terms may be imposed to limit the maximum allowed shift, maximum stretch, and maximum rotations.

Other types of deformation models can be envisioned, such as a standard 3D deformation field. The standard deformation field is estimated between two 3D volumes. A 3D volume is constructed from the SCPR reformatted image cylinder, with voxel values set to 0 outside the coronary cylinder. A rotation to align the frames of the two cylinders may be applied as a preprocessing step before evaluating the full 3D deformation field. The rotation can be estimated from a subset of cylinder frames. The subset may consist of the first j frames, or cylinder frames that have the same anatomical landmarks in both volumes (e.g., calcifications).

Spectral image series produced by some imaging systems may require co-registration i.e., all channels may have to be co-registered to a common reference system. The co-registration can be performed using the deformation model described above. Furthermore, it may be required that the deformation model is robust against failed vessel detections at the distal ends of the vessel. This may be achieved by dampening contributions to the similarity measure of sections of the SCPR images that extend beyond the minimum length of the vessel calculated in native space for each of the vessels.

Examples of an image similarity metric are described next. Various image similarity measures can be used, such as normalized cross correlation (CC), mutual information (MI) metric, etc. The type of image similarity metric may be individualized per channel. The user can visualize the registration result, e.g. as shown in Fig. 8 depicting the correlation between two straightened coronary cylinders. The image gradient schematically represents image intensity variations in the coronary vessels. CCTA scans are typically performed after injection of contrast agent. The amount of contrast agent present in the vessels at the time of scan may vary significantly between different scans (and/or cardiac phase points) of the same patient. Therefore, the similarity measures of spectral channels which are sensitive to contrast agent (in particular virtual mono-energy images corresponding to low keVs, as well as e.g., iodine-density image channels) should not depend on absolute value similarities (such as absolute or squared differences), but rather on information-based similarity measures such as the above-named MI or CC, or geometry-based measures such as derivatives or the above-named local orientation.

Additionally, SCPRs are sensitive to variations in local orientation of the vessel direction. Small changes are amplified in the peripheral regions of each cross-section. At the same time, disease progression or interventions between imaging timepoints increase the chance of image appearance variations inside the vessel or at the vessel wall. Therefore, in-slice spatially adaptive similarity metrics are favored, which de-emphasize similarity mismatches in the vessel and towards the image periphery. One exemplary weighting is given by applying a radially symmetric weighting by a properly scaled and shifted version of the weighting function $f(r) = r^2 \exp(-r), r \geq 0$.

There are multiple further embodiments and variations of the present invention. The proposed algorithm may accept user input for specific segments to guide the registration. A recentering module may be included where the location of the centerline is not assumed to be fully correct but local deformations are allowed. The recentering module operates as an inner loop into the proposed energy minimization scheme. Landmark detectors e.g., for the ostia, stents or specific bifurcation points, may be used to provide constraints for the registration scheme. The algorithm may operate in "tree mode" where the registration is not done between cylindrical segments alone, but the energy function is extended to the entire tree.

The algorithm may operate in a hierarchical fashion, i.e., firstly major vessels are registered, and then smaller vessels are added. If the vessel names are unknown beforehand, the algorithm may be extended by a discrete matching step where candidate segments are matched to each other prior to fine-grained alignment.

Display of the registered SCPR images may include a combination (e.g. overlays) of a functional map (e.g. iodine density) and an anatomical image (e.g. conventional CT), if the functional map is selected in step 106 or 207. A transformed space (from the original spectral channels) can be presented to the user. The visual appearance of that space emphasizes the correspondences/differences.

In summary, according to the present invention, tools are provided to the user to identify which of the multiple channels contains the most significant display of anatomical change by means of analyzing inter-channel variations of the residual image differences after registration. Multi-channel registration is applied to sets of images derived from spectral images, e.g. spectral CT image acquisitions of coronary arteries. Each set of images is acquired at a specific time point (example of time points are time of diagnosis and one or more follow-ups). A set of images may be composed of several virtual monochromatic images (VMIs) corresponding to various levels of X-ray energy, iodine no water image, virtual non-contrast image, effective Z number image, etc. The image contrast (i.e., the degree of grayness, density between image regions) is different across these types of images. For example, high energy VMIs show less image contrast than low energy VMIs. Lesions or calcifications have high intensity values compared with normal tissue on low energy VMIs. Image registration is generally hampered by large intensity variations (such as disease progression between two points in time detected with low energy VMIs).

According to an embodiment a weighted registration approach is presented where different weights are assigned to different VMIs, e.g., higher weights are assigned to higher energy VMIs. A metric quantifying the residual image intensity differences after registration can be used to rank the channels with the most display of anatomical change. Examples of metric: sum of residual differences, maximum, x-th percentile, etc. Additionally, the magnitude of the deformation vector field can be used to show the user the areas of the largest deformations.

While the device has been disclosed as being incorporated into an imaging device, the device may also being provided into a workstation and/or an advanced visualization system configured for receiving spectral image data. For example, such a workstation and/or advanced visualization system may be an on-premises solution and/or a cloud solution.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

Some portions of the description are presented in terms of symbolic representations of operations on non-transient signals stored within a computer memory. These descriptions and representations are used by those skilled in the data processing arts to convey the substance of their work most effectively to others skilled in the art. Such operations typically require physical manipulations of physical quantities. Usually, though not necessarily, these quantities take the form of electrical, magnetic, or optical signals capable of being stored, transferred, combined, compared, and otherwise manipulated. It is convenient at times, principally for reasons of common usage, to refer to these signals as bits, values, elements, symbols, characters, terms, numbers, or the like. Furthermore, it is also convenient at times to refer to certain arrangements of steps requiring physical manipulation of physical quantities as modules or code devices, without loss of generality.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices. The memory may store various software applications including computer executable instructions, that when run on the processor, implement the methods and systems set out herein. Other forms of memory, such as a storage device and a mass storage device, may also be included and accessible by the processor (or processors) via the bus. The storage device and mass storage device can each contain any or all of the methods and systems discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

**Claims**

1. Device for image processing of spectral images of a subject, the device comprising:

   - an input unit (31) configured to obtain two spectral image data sets of a region of interest, ROI, of the subject acquired at different points in time, each spectral image data set comprising two or more spectral images of the ROI in two or more different channels, the ROI including coronaries;
   - a processing unit (32) configured to

     - perform a first alignment of the two spectral image data sets by use of image registration;
     - identify one or more corresponding coronary segments in the aligned spectral image data sets;
     - perform a second alignment of the two spectral image data sets, which is a finer alignment than the first alignment, by use of multi-channel image registration of the one or more corresponding coronary segments; and
   - determine, per channel, image differences between the finer aligned spectral image data sets; and

   - an output unit (33) configured to output the finer aligned spectral images of the channel having the largest image differences.

2. Device according to claim 1, wherein the processing unit (32) is further configured to:

   - generate straightened curved planar reformations for the coronary segments identified in the aligned spectral image data sets; and
   - perform the second alignment of the two spectral image data sets by use of multi-channel image registration of the straightened curved planar reformations of the one or more corresponding coronary segments.

3. Device according to any one of the preceding claims, wherein the processing unit (32) is further configured to perform the multi-channel image registration by minimizing an energy function defined by a weighted sum of similarity measures of each channel.

4. Device according to claim 3, wherein the processing unit (32) is further configured to assign an individual weight to each channel for computing the weighted sum.

5. Device according to claim 4, wherein the processing unit (32) is further configured to use weights that are predetermined for each channel, that are set or modified by a user, or that are automatically computed, in particular by an automatic iterative tweaking method.

6. Device according to any one of claims 3 to 5, wherein the processing unit (32) is further configured to use an individual similarity measure for each channel for computing the weighted sum.

7. Device according to claim 6, wherein the processing unit (32) is further configured to use a similarity measure for each channel selected from a group of similarity measures comprising normalized cross correlation, mutual information metric, information-based similarity measures, geometry-based similarity measures, sum of squared intensity differences, ratio image uniformity, and one or more derivates of a combination of one or more of these metrics.

**8.** Device according to any one of claims 3 to 7, wherein the processing unit (32) is further configured to

- perform the second alignment of the two spectral image data sets multiple times by performing the multi-channel image registration using different combinations of weights and/or similarity measures; and
- determine for each of the second alignments, per channel, image differences between the finer aligned spectral image data sets,

wherein the output unit (33) is configured to output, for each of the second alignments, the finer aligned spectral images of the channel having the largest image differences.

**9.** Device according to any one of claims 3 to 8, wherein the processing unit (32) is further configured to determine, by minimizing the energy function, a deformation model or transformation between spectral images of the same channel from different spectral image data sets, in particular a deformation model or transformation that is inverse consistent or invertible.

**10.** Device according to any one of the preceding claims, wherein the processing unit (32) is configured to determine, per channel, image differences between the finer aligned spectral image data sets by use of a difference metric qualifying residual image intensity differences, in particular a sum of residual differences, a maximum, an x-th percentile, number of voxels in one or more regions defined by connected voxels with image difference above a threshold value, and one or more derivates of a combination of one or more of these metrics.

**11.** Device according to any one of the preceding claims, wherein the processing unit (32) is further configured to:

- extract coronary centerlines in the first aligned spectral image data sets;
- identify the one or more corresponding coronary segments in the extracted coronary centerlines; and
generate straightened curved planar reformations for the coronary segments using the extracted coronary centerlines and the original non-reformatted image data sets.

**12.** Device according to any one of the preceding claims, wherein the spectral image data sets are spectral CT image data sets, in particular coronary computed tomography angiography image data sets and/or wherein the spectral image data sets each comprises two or more of a spectral base image, a monoE40KeV image, an iodine map image, and a Z_effective image.

**13.** Imaging system (1) comprising:

- an imaging device (2) configured to acquire spectral image data sets of a region of interest, ROI, of a subject, each spectral image data set comprising two or more spectral images of the ROI in two or more different channels, the ROI including coronaries;
- a device (3) according to any one of the preceding claims for image processing of the acquired spectral images of the subject; and
- a display unit (4) configured to display finer aligned spectral images of the channel having the largest image differences.

**14.** Method for image processing of spectral images of a subject, the method comprising:

- obtaining two spectral image data sets of a region of interest, ROI, of the subject acquired at different points in time, each spectral image data set comprising two or more spectral images of the ROI in two or more different channels, the ROI including coronaries;
- performing a first alignment of the two spectral image data sets by use of image registration;
- identifying one or more corresponding coronary segments in the aligned spectral image data sets;
- performing a second alignment of the two spectral image data sets, which is a finer alignment than the first alignment, by use of multichannel image registration of the one or more corresponding coronary segments;
- determining, per channel, image differences between the finer aligned spectral image data sets; and
- outputting the finer aligned spectral images of the channel having the largest image differences.

**15.** Computer program comprising program code means for causing a computer to carry out the steps of the method as claimed in claim 14 when said computer program is carried out on the computer.

FIG.1

FIG.2

EP 4 550 264 A1

**FIG.3**

obtain spectral image data sets ～101

perform a first alignment ～102

identify corresponding coronary segments ～103

perform a second alignment ～104

determine image differences ～105

output the finer aligned spectral images ～106

100

**FIG.4**

coarse alignment of image series A and image series B ～201

extract coronary centerlines ～202

identify coronary segments ～203

generate SCPRs for corresponding coronary segments, e.g., for c1 ～204

weighted multichannel registration of SCPR_A and SCPR_B ～205

channel-wise image difference of aligned images and image difference metrics M ～206

rank the channels based on image difference metric M; show result to user ～207

200

EP 4 550 264 A1

series A

$A_1$ $A_2$ $\cdots$ $A_N$

FIG.5A

series B

$B_1$ $B_2$ $\cdots$ $B_N$

centerlines coronary tree A

FIG.5B

centerlines coronary tree B

labeled coronary tree A

c2
c3
c1
c4
c5

FIG.5C

labeled coronary tree B

c2
c3
c1
c4

SCPR for c1 series A

SCPR_$A_1$   SCPR_$A_2$  $\cdots$  SCPR_$A_N$

SCPR for c1 series B

SCPR_$B_1$   SCPR_$B_2$  $\cdots$  SCPR_$B_N$

FIG.5D

EP 4 550 264 A1

SCPR for c1 series A

SCPR_A$_1$   SCPR_A$_2$  ···  SCPR_A$_N$

SCPR for c1 series B

SCPR_B$_1$   SCPR_B$_2$  ···  SCPR_B$_N$

FIG.6

FIG.7

FIG.8

Europäisches
Patentamt
European
Patent Office
Office européen
des brevets

# EUROPEAN SEARCH REPORT

Application Number

EP 23 20 7342

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/097833 A1 (RAZETO MARCO [GB] ET AL) 9 April 2015 (2015-04-09) * paragraph [0016] – paragraph [0082]; figure 5 * | 1-15 | INV. G06T7/33 G06T7/00 A61B6/03 A61B6/50 |
| A | LU GUOLAN ET AL: "Medical hyperspectral imaging: a review", JOURNAL OF BIOMEDICAL OPTICS, SPIE, 1000 20TH ST. BELLINGHAM WA 98225-6705 USA, vol. 19, no. 1, 1 January 2014 (2014-01-01), page 10901, XP060047195, ISSN: 1083-3668, DOI: 10.1117/1.JBO.19.1.010901 [retrieved on 2014-01-20] * Section 4.1.2, last paragraph; page 7, column 2 * | 1-15 | |
| A | CN 112 508 875 A (XIAN KERUISHENG INNOVATIVE TECH CO LTD) 16 March 2021 (2021-03-16) * page 16 * | 1-15 | |

TECHNICAL FIELDS
SEARCHED (IPC)

A61B
G06T

The present search report has been drawn up for all claims

| Place of search | Date of completion of the search | Examiner |
|---|---|---|
| The Hague | 17 April 2024 | Lauritzen, Jan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 20 7342

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2024

| Patent document cited in search report | | Publication date | Patent family member(s) | | Publication date |
|---|---|---|---|---|---|
| US 2015097833 | A1 | 09-04-2015 | CN | 104517303 A | 15-04-2015 |
| | | | JP | 6448972 B2 | 09-01-2019 |
| | | | JP | 2015073901 A | 20-04-2015 |
| | | | US | 2015097833 A1 | 09-04-2015 |
| CN 112508875 | A | 16-03-2021 | CN | 112508875 A | 16-03-2021 |
| | | | CN | 114240841 A | 25-03-2022 |

For more details about this annex : see Official Journal of the European Patent Office, No. 12/82

18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIM, I. Y.** ; **DE WEEK, O. L.** Adaptive weighted sum method for multiobjective optimization: a new method for Pareto front generation. *Structural and Multidisciplinary Optimization.*, 2005, vol. 31 (2), 105-116

- **BARDERA, A.** ; **FEIXAS, M.** ; **BOADA, I.** Normalized similarity measures for medical image registration. *Medical Imaging 2004: Image Processing*, 2004, vol. 5370, 108-118